# EUROPEAN PATENT SPECIFICATION

(11) **EP 1 309 529 B1**
(45) Date of publication and mention of the grant of the patent: **01.12.2004**
(21) Application number: 01956673.6
(22) Date of filing: 10.08.2001
(51) Int. Cl.: C07C 13/20, C07C 13/19, A61K 7/46, C07C 35/08

(54) **FRAGRANCE COMPOUNDS**
RIECHSTOFFE
COMPOSES POUR PARFUMS

(30) Priority: 14.08.2000 EP 00306941
(43) Date of publication of application: 14.05.2003
(73) Proprietor: Quest International Services B.V., 1411 GP Naarden (NL)
(72) Inventor: ELLWOOD, Simon, Ashford, Kent TN25 6QE (GB); HAINES, Jan Thomas, Ashford, Kent TN23 4XT (GB); NEWMAN, Christopher Paul, Canterbury, Kent CT4 7BS (GB)
(74) Representative: Matthews, Heather Clare
(86) International application number: PCT/GB2001/003614
(87) International publication number: WO 2002/014243

(56) References cited:
- WO-A-98/47842

## Description

### Field of the Invention

This invention concerns novel fragrance compounds, their method of production, and their use in perfumes and perfumed products and as intermediates in production of other materials, particularly other fragrance compounds.

### Summary of the Invention

In one aspect the invention provides an alkene having the structure

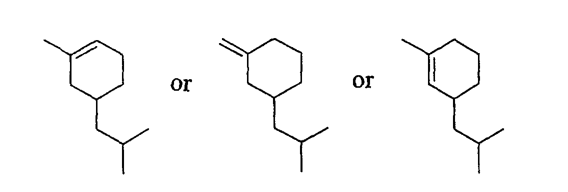

For brevity and simplicity, such materials are referred to herein as the "alkene" or "alkenes", the "novel alkene" or "novel alkenes" or the "alkene of the invention" or "alkenes of the invention".

The alkenes of the invention comprise three isomers, 5-(2-methylpropyl)-1-methyl-1-cyclohexene, 1-methylidene-3-(2-methylpropyl) cyclohexane and 3-(2-methylpropyl)-1-methyl-1-cyclohexene, respectively, and the invention includes within its scope each individual isomer and also mixtures of two or three of the isomers.

The alkenes of the invention, individually and collectively, exhibit interesting fragrance properties or odour characteristics, generally floral, particularly rose and peonile in nature, and so may be used as such to impart, strengthen or improve the odour of a wide variety of products, or may be used as a component of a perfume (or fragrance composition) to contribute its odour character to the overall odour of such perfume. For the purposes of this invention a perfume is intended to mean a mixture of fragrance materials, if desired mixed with or dissolved in a suitable solvent or mixed with a solid substrate, which is used to impart a desired odour to the skin and/or any product for which an agreeable odour is indispensable or desirable. Examples of such products are: fabric washing'powders, washing liquids, washing tablets, fabric softeners and other fabric care products; detergents and household cleaning, scouring and disinfection products; air fresheners, room sprays and pomanders; soaps, bath and shower gels, shampoos, hair conditioners and other personal cleansing products; cosmetics such as creams, ointments, toilet waters, preshave, aftershave, skin and other lotions, talcum powders, body deodorants and antiperspirants.

Other fragrance materials which can be advantageously combined with an alkene according to the invention in a perfume are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic and heterocyclic compounds.

Such fragrance materials are mentioned, for example, in S. Arctander, Perfume and Flavor Chemicals (Montclair, NJ., 1969), in S. Arctander, Perfume and Flavor Materials of Natural Origin (Elizabeth, N.J., 1960) and in "Flavor and Fragrance Materials - 1991", Allured Publishing Co. Wheaton, Ill. USA.

Examples of fragrance materials which can be used in combination with an alkene according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)-propanal, 2,4-dimethylcyclohex-3-enyl-carboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decen-1-ol, phenoxyethyl isobutyrate, phenylacetaldehyde dimethyl acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate.

Solvents which can be used for perfumes which contain an alkene according to the invention are, for example: ethanol, isopropanol, diethyleneglycol monoethyl ether, dipropylene glycol, diethyl phthalate, triethyl citrate, isopropyl myristate.

The quantities in which an alkene according to the invention can be used in perfumes or in products to be perfumed may vary within wide limits and depend, inter alia, on the nature of the product, on the nature and the quantity of the other components of the perfume in which the alkene is used and on the olfactive effect desired. It is therefore only possible to specify wide limits, which, however, provide sufficient information for the specialist in the art to be able to use the alkene according to the invention for his specific purpose. In perfumes an amount of 0.01 % by weight or more of the alkene according to the invention will generally have a perceptible olfactive effect. Typically, the amount of the alkene according to the invention in a perfume is from 0.01% to 25% by weight, preferably from 1% to 5% by weight. The amount of the alkene according to the invention present in products will generally be from 1 to 10,000 ppm by weight, preferably from 10 to 1,000 ppm, depending on the product to be perfumed.

In a further aspect the invention thus provides a perfume comprising an alkene of the invention in an olfactively effective amount.

The invention also covers a perfumed product comprising an alkene of the invention.

The alkenes of the invention are also useful as intermediates in the preparation of other compounds, particularly other fragrance compounds. The alkenes find particular application in the production of 3-(2-methylpropyl)-1-methylcyclohexanol (also referred to as 1-methyl-3-(2-methylpropyl)cyclohexan-1-ol) (as described in WO 98/47842), which is a very useful fragrance material, e.g. by hydration of one or more of the alkenes.

Thus, in a further aspect, the invention includes within its scope a method of making a cycloalkanol, comprising hydrating an alkene of the invention to produce one or more cycloalkanols. Preferably, the cycloalkanol is 3-(2-methylpropyl)-1-methylcyclohexanol.

An alkene in accordance with the invention may conveniently be prepared by an elimination reaction performed on 4-(2-methylpropyl)-1-methoxy-2-methylcyclohexane, 4-(2-methylpropyl)-2-methylcyclohexanol, or a mixture thereof. Preferably, the alkenes of the invention are prepared by an elimination reaction performed on a mixture of 4-(2-methylpropyl)-1-methoxy-2-methylcyclohexane (novel saturated methyl ethers) and 4-(2-methylpropyl)-2-methylcyclohexanol (also known as 4-isobutyl-2-methylcyclohexanol) (known alcohols as disclosed in WO 99/55811), e.g. using 4-methylbenzenesulphonic acid (pTSA) catalyst and cyclohexane solvent. This reaction results in production of a mixture of the three isomeric alkenes of the invention. A suitable mixture of ethers and alcohols for reaction may conveniently be made by catalytic hydrogenation of 1-(4-methoxy-3-methylphenyl)-2-methyl-1-propanone to give 4-(2-methylpropyl)-1-methoxy-2-methylbenzene and then a mixture of the ethers and alcohols.

The resulting isomeric mixture may be used as such, either as a fragrance material or as an intermediate in preparation of other compounds. Alternatively the isomers may be separated, e.g. using known separation techniques such as chromatographic or distillation techniques. For example, 1-methylidene-3-(2-methylpropyl) cyclohexane can be separated from the other two isomers, 5-(2-methylpropyl)-1-methyl-1-cyclohexene and 3-(2-methylpropyl)-1-methyl-1-cyclohexene, by fractional distillation.

The invention will be further described, by way of illustration, in the following Example, which describes a 3 stage process for production of a mixture of alkenes in accordance with the invention, followed by production of 3-(2-methylpropyl)-1-methylcyclohexanol from the mixture of alkenes in a fourth stage.

### Example

GC/GLC conditions utilised for analyses in the following Example:

| | |
|---|---|
| GC | Hewlett Packard HP 6890 Gas Chromatograph |
| Column | HP-5 (SE54) 30m x 0.32mm (internal diameter) x 0.25µm df supplied by Hewlett Packard |
| Carrier Gas | Hydrogen |
| Solvent/Injection Volume | Acetone, 0.2µl |
| Injector | 220°C, split 60:1 |
| Detector | FID, 280°C |
| Temperature Prog. | 70°C (initial oven temperature), hold for 3 minutes, ramp at 10°C/min to 100°C, then ramp at 25°C/min to 280°C and bold for 4 minutes. |

### Stage 1. Friedel Crafts Acylation.

### Experimental

1-methoxy-2-methylbenzene (methyl anisole) (261 kg, 98.6% pure, 2.109 kmol) and trifluoromethanesulphonic acid (triflic acid) (326g, 2.17 mol) (catalyst) were charged to a 1360 litre general purpose glass-lined reactor. The mixture was heated under nitrogen with stirring to a temperature of 150°C. Once at temperature, 1-methylpropanoic anhydride (365 kg, 2.31 kmol) was charged gradually to the reactor over a period of two hours while maintaining the reaction temperature at 150°C. The mixture was stirred for a further one and a half hours at this temperature. After cooling to 40°C the catalyst was neutralised with solid sodium carbonate (230 g, 2.16 mol) and the 2-methylpropanoic acid by-product was distilled off under reduced pressure (30 mBar) up to a pot temperature of 120°C. The resultant material was washed twice with a 5% w/w sodium carbonate solution (60 kg) to leave the crude product, 1-(4-methoxy-3-methylphenyl)-2-methyl-1-propanone, 93.9% pure by GC rpa. (385 kg, 1.88 kmol, 89.2% theoretical yield by analysis based on 1-methoxy-2-methylbenzene).

### Distillation

The crude material (381.4 kg, 1.86 kmol) was purified by distillation under reduced pressure through a 5 theoretical plate packed column. The product, 1-(4-methoxy-3-methylphenyl)-2-methyl-1-propanone (345.5 kg, purity 97.7% rpa, 1.76 kmol) was collected at 137-140°C at 8mBar and crystallised on standing with a melting point of 22 °C. Thus, the overall yield of distilled product was 84.1% based on 1-methoxy-2-methylbenzene.

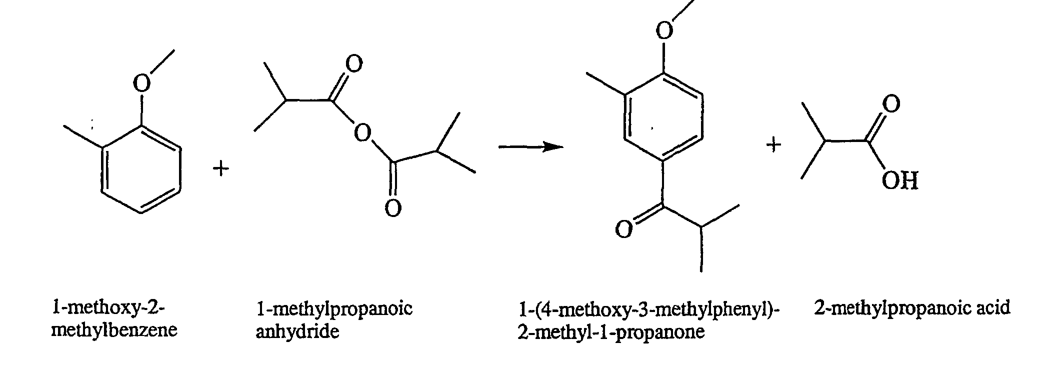

### Stage 2. Hydrogenation

### Experimental

1-(4-methoxy-3-methylphenyl)-2-methyl-1-propanone (60.0 kg, 97.3% pure by GC, 0.304 kmol) made as described above was charged to a high-pressure 100 litre reactor fitted with a stirrer and hydrogen supply. Distilled water (25.0 kg, 1.389 kmol), lactic acid cocatalyst, 85 % aq. solution (2.0 kg, 18.9 mol) and hydrogenation catalyst (5 % palladium on charcoal Type 87L paste ex Johnson Matthey, 1.2 kg) were charged to the reactor. After purging with nitrogen and hydrogen, for the first stage of hydrogenation, the stirring mixture was placed under a hydrogen atmosphere of 20 bar and taken to a temperature of 100°C over a period of 1 hr, maintaining the pressure of 20 bar gauge with the addition of more hydrogen gas. This temperature and pressure was maintained for a further hour until hydrogen addition effectively ceased (< 15 g/hr). For the second stage of hydrogenation, the pressure was raised to 48 bar gauge before heating to 140°C-150°C. This temperature and pressure was maintained for about 6-10 hours (with the reaction time depending on the quality of both the catalyst and feedstock) until hydrogen addition effectively ceased. After cooling to room temperature the pressure was released and, after nitrogen purging, the mixture was filtered to remove the hydrogenation catalyst. The aqueous layer was separated from the crude product (55.4 kg).

Detailed GLC analysis of the crude product showed an isomeric mixture of 4-(2-methylpropyl)-1-methoxy-2-methylcyclohexanes and 4-(2-methylpropyl)-2-methylcyclohexanols constituting 93.3 % rpa of the product. According to this analysis, these desired products totalled 0.29 kmol, which corresponded to a 92.8% theoretical yield based on 1-(4-methoxy-3-methylphenyl)-2-methyl-1-propanone.

### Distillation

Distillation is not essential, but usefully the product ethers and alcohols are together separated from water, light heads and residues by a simple distillation up a short, packed column of about 5 theoretical plates: Typically, crude 4-(2-methylpropyl)-1-methoxy-2-methylcyclohexane/4-(2-methylpropyl)-2-methylcyclohexanol (55.4 kg, 93.3 % rpa pure by GC, 0.29 kmol) yielded an isomeric mixture of 4-(2-methylpropyl)-1-methoxy-2-methylcyclohexanes and 4-(2-methylpropyl)-2-methylcyclohexanols (52.4 kg, 96.0 % rpa pure by GC, 0.28 kmol) having a boiling range of 98-116°C/30 mBar.

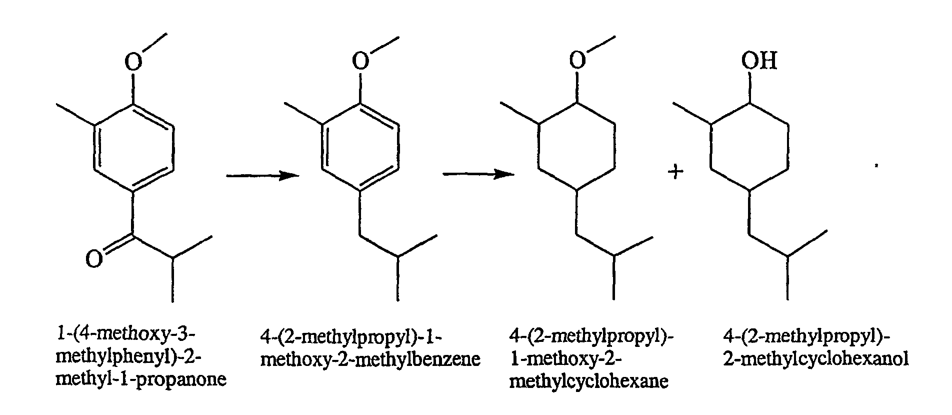

The ethers occur in a number of different isomeric forms as follows:

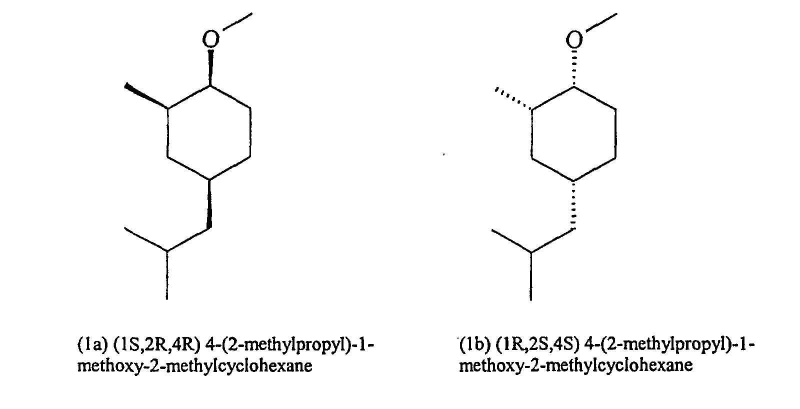

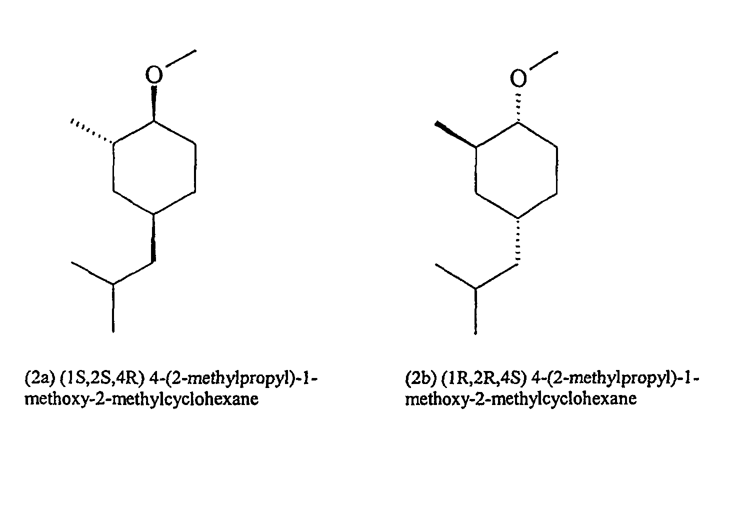

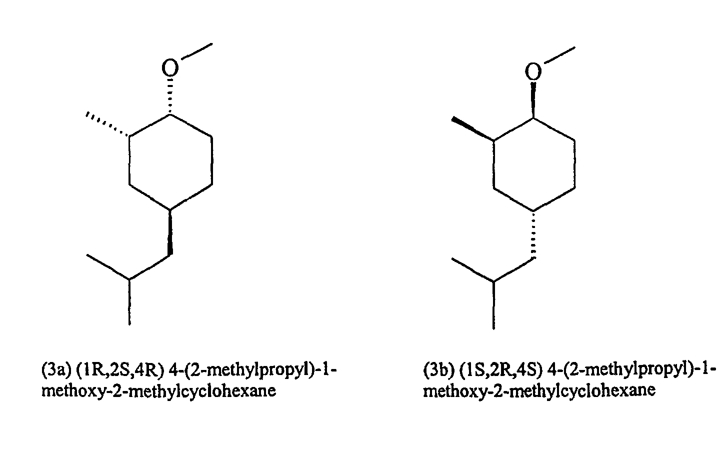

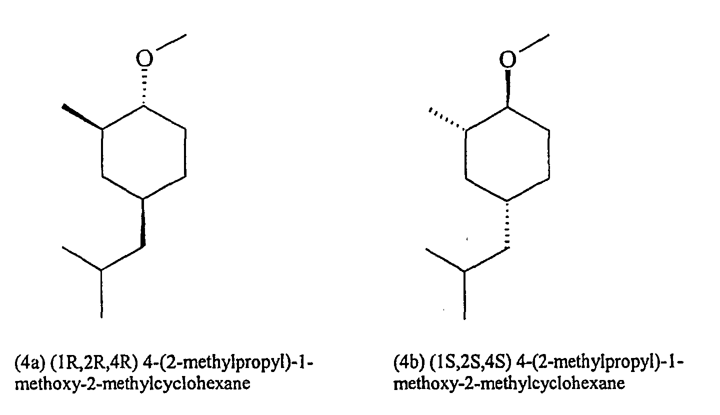

The alcohols occur in a number of corresponding isomeric forms as follows:

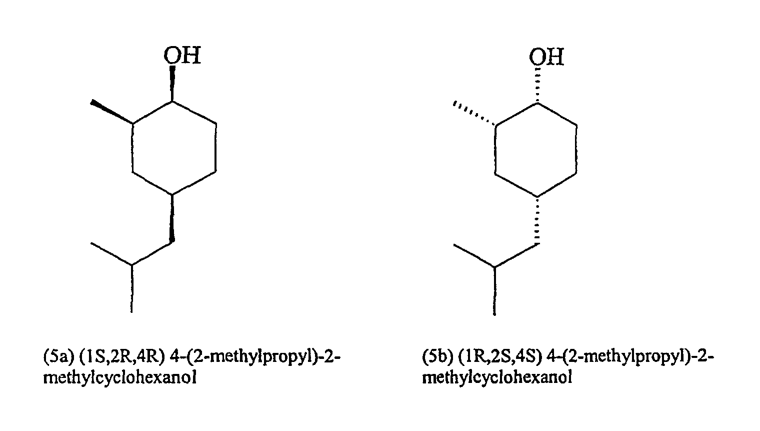

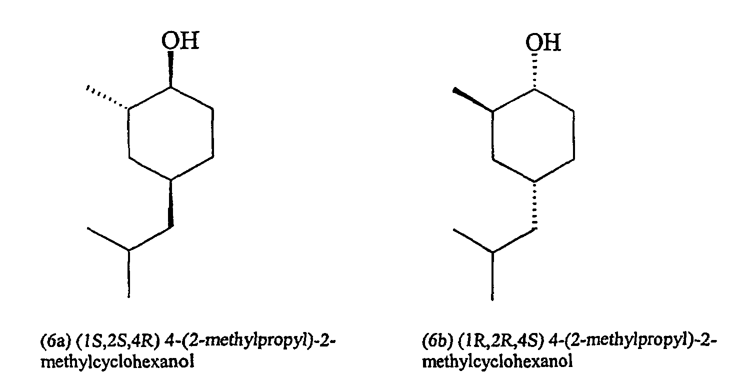

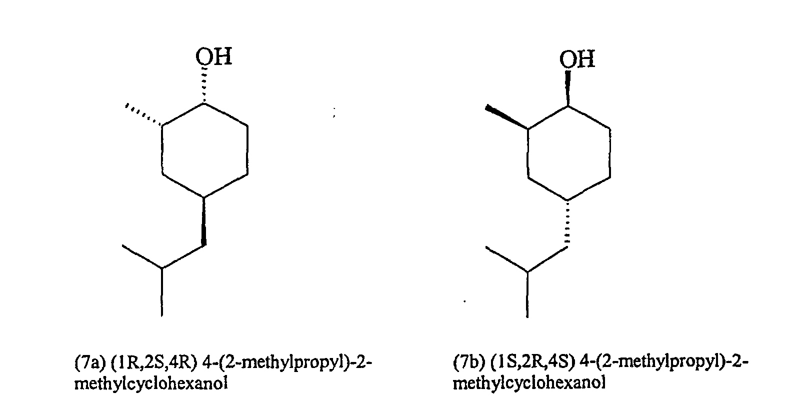

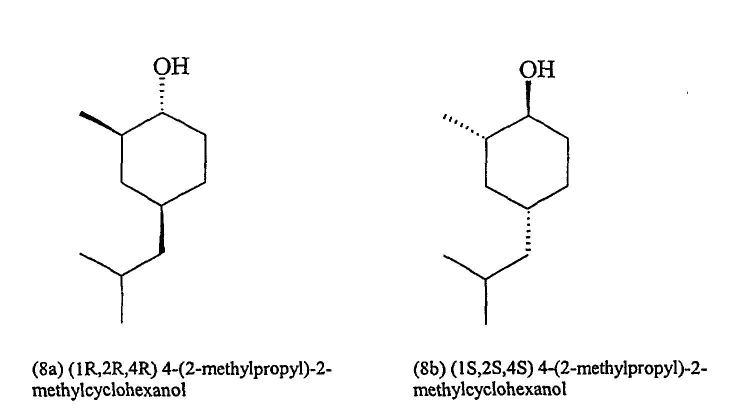

### Stage 3. Elimination

### Experimental

An isomeric mixture of 4-(2-methylpropyl)-1-methoxy-2-methylcyclohexanes and 4-(2-methylpropyl)-2-methylcyclohexanols (46.3 kg, 92.6 % rpa pure by GC, ∼0.24 kmol) made as described above, 4-methylbenzenesulphonic acid monohydrate (pTSA catalyst) (1.4 kg, 7.4 mol) and cyclohexane (10.0 kg) were charged to a 100 litre glass-lined general purpose reactor. The stirred mixture was heated to a temperature of 150°C under a nitrogen atmosphere. The cyclohexane/water/methanol azeotrope was removed in the Dean & Stark, with the upper cyclohexane layer being returned to the flask. The quantity of cyclohexane was adjusted to maintain the flask temperature at 150°C-155°C. These conditions were maintained for 8 hours. After cooling to 40°C the mixture was washed twice with a 5% w/w sodium carbonate solution (9.0 kg) and dehydrated to leave the crude product 3-(2-methylpropyl)-1-methyl-1-cyclohexene/5-(2-methylpropyl)-1-methyl-1-cyclohexene (typically in the ratio 1:2), 40.4 kg, 66.9 % rpa pure by GC (27.0 kg, 0.18 kmol) with trace amounts of 1-methylidene-3-(2-methylpropyl)cyclohexane, and unreacted starting materials 9.6 % rpa by GC (3.9 kg, 0.02 kmol). Thus, the chemical yield of this reaction was 75 % and the selectivity was 82 %.

### Distillation

The crude product (80.4 kg) from two such reactions, containing 64.5 % rpa pure by GC (0.34 kmol) of the desired alkenes was purified by careful fractional distillation under reduced pressure through a 30 theoretical plate packed column at a 20:1 reflux ratio. The mixture of 3-(2-methylpropyl)-1-methyl-1-cyclohexene and 5-(2-methylpropyl)-1-methyl-1-cyclohexene was collected in the range 88-93°C/32-40mBar. The purified product (61 kg) contained 81.5 % rpa of the desired alkenes corresponding to a distillation yield of 96 %. The product is a liquid at normal temperatures.

The unreacted starting materials remained in the distillation residues.

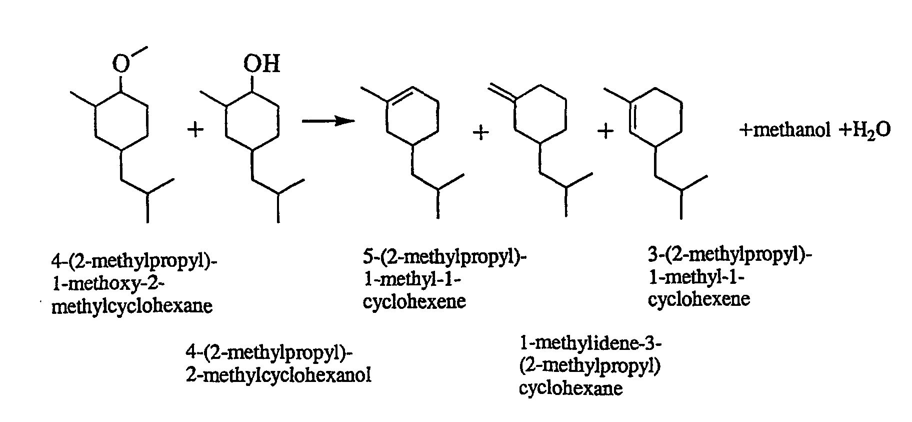

### Stage 4. Hydration

### Experimental

A mixture of 3-(2-methylpropyl)-1-methyl-1-cyclohexene and 5-(2-methylpropyl)-1-methyl-1-cyclohexene (30.1 kg, "purity" 82.1% rpa, 0.16 kmol) together with a small amount of 1-methylidene-3-(2-methylpropyl)cyclohexane, made as described above was charged to a 100 litre glass-lined general purpose reactor. 3-(2-methylpropyl)-1-methylcyclohexanol (0.3 kg, 1.76 mol) was added (this small amount of the desired end product acts as a phase transfer agent and helps the reaction initiate). The stirred mixture was cooled to -5°C and sulphuric acid, 76% w/w (20.4 kg, 0.158 kmol) was added slowly over 5 hours, maintaining the temperature in the range -6°C to -3°C. The stirred mixture was held at this temperature range for a further hour after the acid addition. The mixture was slowly added to water (80.0 kg, 4.4 kmol) in a well-stirred quench vessel maintaining the temperature below 25°C, before separating the lower aqueous phase. The organic layer was washed with water (11.0 kg) while still maintaining the temperature below 25 °C. The residual acid catalyst was neutralised by washing the reaction mixture with 5% sodium hydroxide solution (10.0 kg), maintaining a temperature below 30°C. The resultant product was washed twice with water (10.0 kg) at 50°C - 55°C to leave the crude product (3-(2-methylpropyl)-1-methylcyclohexanol) (30.8 kg). GLC analysis showed that this crude product contained 52.55% rpa (0.095 kmol) of pure 3-(2-methylpropyl)-1-methylcyclohexanol, in the form of a mixture of trans and cis isomers. This corresponds to a chemical yield of 59.1 % based on 3-(2-methylpropyl)-1-methyl-1-cyclohexene/5-(2-methylpropyl)-1-methyl-1-cyclohexene.

### Distillation

The crude product (30.8 kg, 0.095 kmol) was purified by fractional distillation under reduced pressure through a packed column of about 15 theoretical plates. The product (3-(2-methylpropyl)-1-methylcyclohexanol (15.28 kg, 98.7 % by GC rpa, 0.089 kmol) was collected at 98-100°C, 15 mBar. This gave an overall yield of 55.1 %w/w of Perfumery Quality material based on 3-(2-methylpropyl)-1-methyl-1-cyclohexene/5-(2-methylpropyl)-1-methyl-1-cyclohexene. The product, 3-(2-methylpropyl)-1-methylcyclohexanol was a mixture of approximately 35-45% cis, and about 55-65% trans isomers. The product may solidify on standing with a melting point of 20-25°C.

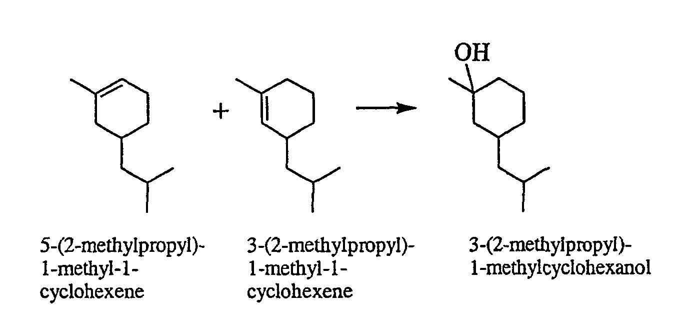

## Claims

1. An alkene having the structure

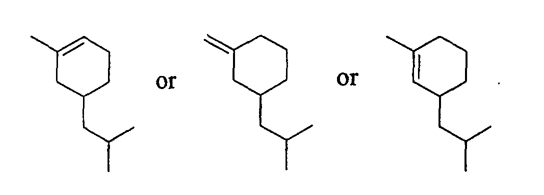

2. An alkene according to claim 1, comprising an individual isomer.

3. An alkene according to claim 1, comprising a mixture of two or more of the isomers.

4. An alkene according to claim 1, 2 or 3, made by an elimination reaction performed on 4-(2-methylpropyl)-1-methoxy-2-methylcyclohexane, 4-(2-methylpropyl)-2-methylcyclohexanol, or a mixture thereof.

5. An alkene according to any one of the preceding claims, made by an elimination reaction performed on a mixture of 4-(2-methylpropyl)-1-methoxy-2-methylcyclohexane, and 4-(2-methylpropyl)-2-methyl-cyclohexanol.

6. A perfume comprising an alkene in accordance with any one of the preceding claims in an olfactively effective amount.

7. A perfumed product comprising an alkene according to any one of claims 1 to 5 or a perfume according to claim 6.

8. A method of making a cycloalkanol, comprising hydrating an alkene in accordance with any one of claims 1 to 5 to produce one or more cycloalkanols.

9. A method according to claim 8, wherein the cycloalkanol is 3-(2-methylpropyl)-1-methylcyclohexanol.

## Patentansprüche

1. Alken, das die Struktur 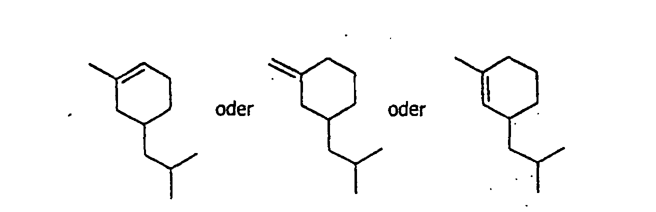 hat.

2. Alken nach Anspruch 1, das ein einzelnes Isomer umfasst.

3. Alken nach Anspruch 1, das ein Gemisch aus zwei oder mehreren der Isomeren umfasst.

4. Alken nach Anspruch 1, 2 oder 3, das durch eine Eliminierungsreaktion hergestellt wurde, welche an 4-(2-Methylpropyl)-1-methoxy-2-methylcyclohexan, 4-(2-Methylpropyl)-2-methylcyclohexanol oder einem Gemisch davon durchgeführt wurde.

5. Alken nach einem der vorangehenden Ansprüche, das durch eine Eliminierungsreaktion hergestellt wurde, welche an einem Gemisch aus 4-(2-Methylpropyl)-1-methoxy-2-methylcyclohexan und 4-(2-Methylpropyl)-2-methyl-cyclohexanol durchgeführt wurde.

6. Parfüm, umfassend ein Alken nach einem der vorangehenden Ansprüche, in einer olfaktiv wirksamen Menge.

7. Parfümprodukt, das ein Alken nach einem der Ansprüche 1 bis 5 oder ein Parfüm nach Anspruch 6 umfasst.

8. Verfahren zur Herstellung eines Cycloalkanols, das Hydratisieren eines Alkens nach einem der Ansprüche 1 bis 5 unter Herstellung eines oder mehrerer Cycloalkanole umfasst.

9. Verfahren nach Anspruch 8, wobei das Cycloalkanol 3-(2-Methylpropyl)-1-methylcyclohexanol ist.

## Revendications

1. Alcène ayant la structure

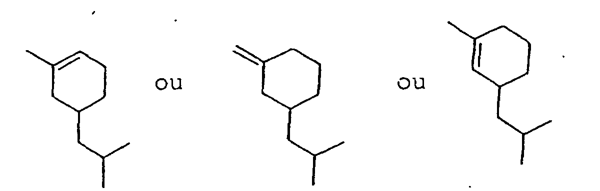

2. Alcène selon la revendication 1, comprenant un isomère individuel.

3. Alcène selon la revendication 1, comprenant un mélange de deux ou plusieures des isomères.

4. Alcène selon la revendication 1, 2 ou 3, préparé au moyen d'une réaction d'élimination effectuée sur du 4-(2-méthylpropyl)-1-méthoxy-2-méthylcyclohexane, du 4-(2-méthylpropyl)-2-méthylcyclohexanol ou un mélange de ceux-ci.

5. Alcène selon l'une quelconque des revendications précédentes, préparé au moyen d'une réaction d'élimination effectuée sur un mélange de 4-(2-méthylpropyl)-1-méthoxy-2-méthylcyclohexane et de 4-(2-méthylpropyl)-2-méthylcyclohexanol.

6. Parfum comprenant un alcène selon l'une quelconque des revendications précédentes en une quantité efficace du point de vue olfactif.

7. Produit parfumé comprenant un alcène selon l'une quelconque des revendications 1 à 5 ou un parfum selon la revendication 6.

8. Procédé de préparation d'un cycloalcanol, comprenant l'hydratation d'un alcène selon l'une quelconque des revendications 1 à 5 pour produire un ou plusieurs cycloalcanols.

9. Procédé selon la revendication 8, dans lequel le cycloalcanol est du 3-(2-méthylpropyl)-1-méthylcyclohexanol.
